# EUROPÄISCHE PATENTSCHRIFT

(11) **EP 3 559 921 B1**
(45) Veröffentlichungstag und Bekanntmachung des Hinweises auf die Patenterteilung: **25.09.2024**
(21) Anmeldenummer: 17811446.8
(22) Anmeldetag: 29.11.2017
(51) Int. Cl.: A61B 5/11, A61B 5/00, G01D 4/00, G08B 21/04, G08B 25/00

(54) **VERFAHREN UND VORRICHTUNG ZUR AKTIVITÄTSÜBERWACHUNG WENIGSTENS EINER PERSON IN EINER INFRASTRUKTUREINHEIT UND MESSEINRICHTUNG**
METHOD AND APPARATUS FOR MONITORING THE ACTIVITY OF AT LEAST ONE PERSON IN AN INFRASTRUCTURE UNIT, AMD MEASURING DEVICE
PROCÉDÉ ET DISPOSITIF POUR SURVEILLER LES ACTIVITÉS D'AU MOINS UNE PERSONNE DANS UNE UNITÉ D'INFRASTRUCTURE, ET DISPOSITIF DE MESURE

(30) Priorität: 21.12.2016 DE 102016015247; 31.01.2017 DE 102017000838
(43) Veröffentlichungstag der Anmeldung: 30.10.2019
(73) Patentinhaber: Diehl Metering GmbH, 91522 Ansbach (DE)
(72) Erfinder: SCHULZE, Manfred, 91522 Ansbach (DE); WESTPHAL, Robert, 88239 Wangen (DE)
(74) Vertreter: Diehl Patentabteilung
(86) Internationale Anmeldenummer: PCT/EP2017/001388
(87) Internationale Veröffentlichungsnummer: WO 2018/114035

(56) Entgegenhaltungen:
- EP-A1- 2 822 225
- EP-A2- 1 349 128
- US-A1- 2006 261 962
- US-A1- 2007 152 837

## Beschreibung

Die Erfindung betrifft ein Verfahren zur Aktivitätsüberwachung wenigstens einer Person in einer Infrastruktureinheit, die mit Verbrauchsmedien, umfassend wenigstens Strom und Wasser, versorgt wird, wobei jedem Verbrauchsmedium eine Messeinrichtung zur Messung eines Verbrauchswerts des Verbrauchsmediums zugeordnet ist. Daneben betrifft die Erfindung eine entsprechende Vorrichtung und eine Messeinrichtung.

Bei älteren, pflegebedürftigen oder sicherheitsorientierten Personen ist es bekannt, deren Aktivität zu überwachen, um Änderungen in deren Gesundheitszustand automatisiert feststellen zu können. Eine derartige Aktivitätsüberwachung wird häufig auch als "Aktivitätsmonitoring" bezeichnet. Die Zahl der pflegebedürftigen und/oder alleine lebenden Menschen wächst zurzeit und nach Prognosen auch in der Zukunft erheblich. Unfälle im Haus und verschlechternde Gesundheitszustände stellen somit ein erhebliches Lebensrisiko dar.

Dabei existieren bereits eine Vielzahl von Ansätzen, um Personen in Wohnungen oder Häusern, allgemein in Infrastruktureinheiten, Sicherheit durch Aktivitätsüberwachung zu geben. So ist es beispielsweise bekannt, dass ein täglicher, persönlicher Besuch eines Dienstleisters stattfindet, was jedoch meist sehr aufwändig und teuer ist. Bekannt ist es zudem, Notrufeinrichtungen zu verwenden, die einen Notrufknopf oder einen Bestätigungsknopf aufweisen, wobei derartige Notrufeinrichtungen insbesondere am Körper getragen werden können. Bekannt ist es auch, derartige Notrufeinrichtungen in der Infrastruktureinheit, insbesondere einer Wohnung oder einem Haus, vorzusehen, wobei unterschiedliche Bedienkonzepte für derartige Notrufeinrichtungen bestehen. So kann eine Notrufeinrichtung mit einem Notrufknopf, die am Körper getragen wird, dann zur Betätigung vorgesehen sein, wenn tatsächlich ein Notfall eintritt. Möglich ist es aber auch, insbesondere bei in der Infrastruktureinheit festinstallierten Notrufeinrichtungen, einen Taster bzw. allgemein ein Bedienelement vorzusehen, das regelmäßig, beispielsweise zweimal täglich, betätigt werden muss, um zu signalisieren, dass alles in Ordnung ist. Derartige Notrufeinrichtungen erfordern jedoch ebenso einen hohen Kosteneinsatz und setzen die Fähigkeit der Person voraus, entsprechende Bedienelemente zu betätigen.

Hinsichtlich des Einsatzes von Sensoren wurde bereits vorgeschlagen, beispielsweise Fußbodensensoren vorzusehen, die als Drucksensoren ausgebildet sein können, um liegende Personen innerhalb der Infrastruktureinheit erkennen zu können. Auch wurden Infrarotsensoren zur Sturzerkennung vorgeschlagen. Auch Bewegungssensoren in Räumen der Infrastruktureinheit können zur Aktivitätsüberwachung eingesetzt werden. Schließlich wurde vorgeschlagen, Stromsensoren an einzelnen Verbrauchern vorzusehen, wenn beispielsweise der entsprechende Verbraucher regelmäßig zu bestimmten Zeiten genutzt wird. Ein Beispiel hierfür ist ein Toaster.

Problematisch an all diesen Möglichkeiten ist, dass zusätzliche, kostenintensive und/oder aufwendige Vorrichtungen benötigt werden, um die Aktivitätsüberwachung zu ermöglichen. Zudem zielen eine Vielzahl der Lösungen nur auf bestimmte Vorkommnisse ab, so dass sie - alleine eingesetzt - nicht immer verlässlich Notfälle erkennen und entsprechend agieren können.

EP 1 349 128 A2 offenbart ein System zur Überwachung einer bewohnten Umgebung. Das Überwachungssystem umfasst dabei ein Steuergerät, welchem als Eingangssignal das Ausgangssignal eines mit einem Messmittel verbundenen Signalgenerators zugeführt wird. Gemäß dem dortigen Ausführungsbeispiel kann es sich bei der überwachten Größe um einen Wasserfluss handeln. Durch das Steuergerät wird bei Benutzung der Wasserverbrauch aufgezeichnet, wobei ein aktueller Wasserverbrauch mit in dem Steuergerät hinterlegten Wasserverbrauchssignaturen verglichen wird. Eine Software analysiert den aktuellen Wasserverbrauch sowie das Muster und die Frequenz des Verbrauchs. Bei Feststellen eines abnormalen Wasserverbrauchs wird ein Alarmsignal erzeugt.

US 2006/0 261 962 A1 beschreibt die Verwendung eines Stromsensors, welcher mit der Stromzuleitung einer Wohnumgebung verbunden ist, zur Ermittlung verschiedener Aktivitäten eines Bewohners. Durch Schwankungen in einer überwachten Größe wie der Stromstärke kann auf das Einschalten verschiedener Geräte zurückgeschlossen werden, so dass verschiedene Arten von Aktivitäten der überwachten Person, welche jeweils auf das Verwenden unterschiedlicher elektrischer Geräte zurückgehen, ermittelt werden können. Bei Feststellung einer ungewöhnlichen Aktivität kann die Benachrichtigung einer externen Stelle vorgenommen werden.

EP 2 822 225 A1 beschreibt eine Recheneinrichtung sowie eine Methode zur Heimautomatisierung beschrieben. Das Heimautomatisierungssystem umfasst dabei verschiedene Geräte und Versorgungsleitungen. Bei den Geräten kann es sich beispielsweise um eine Waschmaschine oder einen Toaster handeln. Weiterhin umfasst das Automatisierungssystem einen Anwesenheitssensor, mittels welchem eine Anwesenheitsinformation erzeugt wird.

US 2007/0152837 A1 beschreibt ein Verfahren und eine Vorrichtung zur Überwachung der Aktivität einer Person. Dabei werden mehrere Sensoren eingesetzt, welche von der zu überwachenden Person während ihrer täglichen Aktivitäten aktiviert werden. Die Aktivierung der Sensoren ist dabei als ein Auslösen eines bereits eingeschalteten Sensors zu verstehen. Die Aktivierung der Sensoren kann dabei mit einem vorgegebenen, zeitabhängigen Aktivierungsmuster verglichen werden, um zu ermitteln, ob eine Alarmgabe an die Person erforderlich ist. In einem Ausführungsbeispiel kann nach der Aktivierung eines ersten Sensors ein Timer gestartet wird, wobei auf die Aktivierung desselben Sensors und/oder eines weiteren Sensors innerhalb eines vorgegebenen Zeitfensters gewartet wird, bevor eine Alarmgabe an die überwachte Person oder an Dritte erfolgt.

Der Erfindung liegt daher die Aufgabe zugrunde, eine Möglichkeit zur verlässlichen, aufwandsarm zu realisierenden Aktivitätsüberwachung für Personen in Infrastruktureinheiten, insbesondere Wohnungen und/oder Häusern, anzugeben.

Zur Lösung dieser Aufgabe sind bei einem Verfahren der eingangs genannten Art erfindungsgemäß die Merkmale des Anspruchs 1 vorgesehen.

Erfindungsgemäß wird mithin vorgeschlagen, Messdaten in Form von Verbrauchswerten in ihrem zeitlichen Verlauf zu analysieren und anhand eines aktuellen Verbrauchsprofils festzustellen, ob eine normale Aktivität der Person vorliegt oder nicht. Bei den Messeinrichtungen kann es sich insbesondere um einen Stromzähler und einen Wasserzähler handeln, wie diese im Stand der Technik grundsätzlich bekannt sind. Diese sind nun ausgebildet, mit einer hinreichend guten Zeitauflösung, beispielsweise wenigstens mit einer Minutenauflösung, Verbrauchswerte auszugeben, aus denen sich entsprechend Verbrauchswertverläufe über die Zeit ergeben. Diese stellen als Profildaten Verbrauchsprofile wenigstens für Wasser und Strom dar. Ein gemessener Verbrauchswert beschreibt dabei bevorzugt einen bei der Messung aktuellen Verbrauch, insbesondere pro Zeiteinheit. Dabei sei angemerkt, dass sich Verbrauchswertverläufe über die Zeit selbstverständlich auch bei akkumulierter Zählung oder dergleichen mit entsprechenden Referenzverläufen vergleichen lassen, wobei gegebenenfalls ein entsprechender Offset berücksichtigt werden muss. In einem solchen Fall liefert jede der Messeinrichtungen mithin regelmäßig, insbesondere zyklisch, als Verbrauchswert einen aktuell akkumulierten Verbrauch.

In einer Speichereinrichtung der das Verfahren durchführenden Vorrichtung liegt nun ein Referenzprofil, beschrieben durch Referenzdaten, vor. Das Referenzprofil deckt dabei alle im Verfahren verwendbaren Zeitdauern ab, so dass unter einem Vergleichsprofil für die Zeitdauer im Rahmen der vorliegenden Erfindung zu verstehen ist, dass das Referenzprofil bevorzugt die komplette Zeit abdeckend vorliegt und der dem Vergleich zugrundeliegenden Zeitdauer entsprechende Anteil der alle denkbaren Zeitdauern abdeckenden Referenzdaten für den Vergleich mit den Profildaten herangezogen wird. Mit anderen Worten werden die dem Vergleich zugrundeliegenden, dem Vergleichsprofil für die Zeitdauer entsprechenden Referenzdaten aus das Referenzprofil für alle verwendbaren Zeitdauern beschreibenden Referenzdaten ausgewählt, so dass ein sinnvoller Vergleich möglich ist.

Das Referenzprofil und somit auch das Vergleichsprofil beschreibt die normale Aktivität der wenigstens einen Person in der Infrastruktureinheit. Werden mithin nun die aktuellen Profildaten mit einem entsprechenden Vergleichsprofil der Referenzdaten verglichen, lässt sich feststellen, ob und insbesondere auch in welchem Maße Abweichungen von der normalen Aktivität vorliegen. Liegt keine normale Aktivität vor, schlägt der Vergleich also fehl, ist davon auszugehen, dass ein kritischer Zustand bestehen kann, weshalb wenigstens eine Alarmmaßnahme durchgeführt wird, beispielsweise eine Anforderung für die Durchführung einer Aktion an die Person selbst, um das Vorliegen eines Notfalls feststellen zu können, oder das Senden von Informationen an weitere Personen oder Einrichtungen.

Die der Erfindung zugrundeliegende Aufgabe wird mithin durch die Nutzung bereits vorhandener Infrastruktur gelöst, so dass keine zusätzlichen Sensoren und/oder Datenkanäle erforderlich sind. Es werden lediglich die zeitlich gemessenen Verbrauchswerte von Wasser und Strom analysiert, und daraus ein normales Verhalten von einem kritischen Zustand unterschieden. Somit kann aufwandsarm eine verlässliche Überprüfung des Aktivitätszustands von Personen erfolgen.

Es werden mithin die ohnehin vorhandenen Verbrauchswerte der Strom- und Wassermesseinrichtungen verwendet, um eine Aktivitätsüberwachung darzustellen. Derartige Messeinrichtungen (insbesondere Stromzähler und Wasserzähler) sind in den entsprechenden Infrastruktureinheiten, insbesondere einem Haus und/oder einer Wohnung, ohnehin installiert, da in vielen Ländern diese Art der Verbrauchsmessung Pflicht ist. Die Messeinrichtungen generieren zeitlich hinreichend hoch aufgelöste Verbrauchswerte, die über eine Datenschnittstelle weitergeleitet werden können. Dabei sei an dieser Stelle darauf hingewiesen, dass die Grundlage der hier beschriebenen Aktivitätsüberwachung selbstverständlich die Datensouveränität des Nutzers, also der Person, ist, das bedeutet, der Datenschutz kann dadurch gewährleistet werden, dass die Verwendung der Verbrauchswerte zum eigenen Wohl zuvor entsprechend vereinbart wird.

Zeitliche Verbrauchswertverläufe für Wasser und Strom zeichnen sich durch regelmäßig bzw. statistisch verteilt wiederkehrende Verbrauchsereignisse aus. So existieren beispielsweise typische Verbrauchsereignisse bezüglich der Höhe und der Zeit für Wasser, insbesondere die Verwendung einer Dusche, einer Toilette, von Handwaschbecken, einer Badewanne, des Spülbeckens, einer Spülmaschine, einer Waschmaschine und der Gartenbewässerung mit Kanne und/oder Schlauch. Für den Strom sind beispielhaft die Verwendung Herd, Backofen, Mikrowelle, Spülmaschine, Waschmaschine, Gefrierschrank, Kühlschranktür, Fernsehgerät, Radio, Computer und Licht zu nennen. Mithin zeigt das normale Leben ein typisches, insbesondere personenabhängiges Profil. Die hierbei auftretenden, wiederkehrenden Muster werden durch die Referenzdaten, mithin das Referenzprofil, beschrieben. Die Vielzahl der Nutzungen ermöglicht es auf besonders verlässliche Weise, schnell eine atypische Situation zu erkennen. Dabei ist besonders vorteilhaft, dass die Verbräuche von Wasser und Strom gemeinsam betrachtet werden, da sie häufig korreliert sind. So stehen beispielsweise bestimmte Handlungen bezüglich Wasser, beispielsweise das Abfüllen von zu kochendem Wasser, oft mit Handlungen bezüglich des Stroms (Anschalten des Herdes oder eines Wasserkochers) mittelbar oder unmittelbar in Verbindung und umgekehrt (Inbetriebnahme einer Spülmaschine und entsprechender Wasserverbrauch). Menschen weisen zudem häufig bestimmte Tagesabläufe auf, beispielsweise der Morgentoilette folgend zu duschen, dann über elektrisch betriebene Vorrichtungen das Frühstück zu bereiten, dann zu duschen, etc., so dass ein zusammenhängendes Gesamtbild bei der Betrachtung von Verbrauchswertverläufen für Strom und Wasser entsteht.

Konkret kann hierbei vorgesehen sein, dass im Rahmen des Vergleichs ein die Übereinstimmung mit dem Vergleichsprofil beschreibender Korrelationswert ermittelt und mit einem Schwellwert verglichen wird. Mit anderen Worten kann mittels eines grundsätzlich bekannten Verfahrens eine Korrelation der Profildaten und der Referenzdaten des Vergleichsprofils durchgeführt werden, konkret insbesondere der Verbrauchswertverläufe. Hierbei können verschiedene Einflussfaktoren stärker oder weniger stark gewichtet werden, nachdem beispielsweise die Zahl der Toilettengänge und/oder Zeitpunkte der Nutzung bestimmter elektrischer Geräte zumindest leicht variieren können, aber beispielsweise innerhalb eines bestimmten Zeitfensters üblicherweise auftreten sollten. Ergebnis der durchgeführten Korrelationsbetrachtung ist ein Korrelationswert, der beschreibt, inwieweit sich aufgrund der Referenzdaten erwartete Verbrauchsstrukturen und -muster innerhalb der Profildaten wiederfinden lassen. Der Schwellwert, mit dem der Korrelationswert verglichen wird, beschreibt dabei letztlich, wie "scharf" das Aktivitätskriterium geschaltet ist; er kann insbesondere in Ausführungsbeispielen der vorliegenden Erfindung auch benutzerseitig anpassbar gestaltet werden. Liegt der Korrelationswert oberhalb des Schwellwerts, ist davon auszugehen, dass eine normale Aktivität vorliegt; liegt er unterhalb des Schwellwerts, liegt eine anormale Aktivität vor, so dass entsprechend die wenigstens eine Alarmmaßnahme durchgeführt wird.

In besonders bevorzugter Ausgestaltung der vorliegenden Erfindung werden personenspezifische Referenzdaten ermittelt, insbesondere in einer Lernphase durch Auswertung von Profildaten der Person als Trainingsdaten in der Lernphase mittels eines Lernalgorithmus der künstlichen Intelligenz und/oder der statistischen Datenauswertung. Personen unterscheiden sich häufig in der Art, Abfolge und den Zeitpunkten ihrer regelmäßig wiederkehrenden Tätigkeiten, insbesondere also auch bei solchen, die Stromverbrauch und/oder Wasserverbrauch mit sich bringen. Die Referenzdaten werden also bevorzugt auf die wenigstens eine zu überwachende Person bezogen ermittelt und beschreiben deren normale, personenabhängige Aktivität. Bevorzugt wird das Referenzprofil, aus dem die Vergleichsprofile für die verwendbaren Zeitdauern abgeleitet werden können, in einer Selbstlernphase ermittelt, wobei beispielsweise mithilfe von "smart data analytics" aus den Trainingsdaten, als Profildaten der Lernphase ermittelt, Aktivitätswissen generiert werden kann. Dabei werden bevorzugt Algorithmen der künstlichen Intelligenz verwendet, um ein sogenanntes Maschinenlernen ("machine learning") zu realisieren. Der Begriff der "smart data analytics" setzt sich dabei zusammen aus "smart data" für strukturiert vorliegendes Trainingswissen und "analytics" für Maschinenlerntechniken, insbesondere unter Verwendung künstlicher Intelligenz, wie beispielsweise eines neuronalen Netzwerks. Doch auch statistische Methoden der Datenanalyse können sich selbstverständlich zusätzlich oder alternativ eingesetzt werden, um ein Maschinenlernen aufgrund von Trainingsdaten zu ermöglichen. Ergebnis können als Referenzprofil beispielsweise mittlere Verbrauchswertverläufe für die wenigstens eine Person sein, denen jeweils Abweichungsinformationen über üblicherweise auftretende Abweichungen und deren Rahmen zugeordnet sind.

In diesem Kontext ist es zweckmäßig, wenn bei erfülltem Aktivitätskriterium die Referenzdaten unter Nutzung der der Erfüllung zugrundeliegenden Profildaten aktualisiert werden. Mit anderen Worten kann eine ständige Aktualisierung und sozusagen "Nachführung" des Referenzprofils und somit der Referenzdaten erfolgen, um sie beispielsweise Veränderungen der Lebensgewohnheiten anpassen zu können und dergleichen.

Zweckmäßig ist es in diesem Kontext ferner, wenn die Referenzdaten abhängig von der Personenzahl in der Infrastruktureinheit und/oder von dem Kalendertag ermittelt werden, wobei für den Vergleich im Aktivitätskriterium auf die aktuelle Personenzahl und/oder den aktuellen Kalendertag bezogene Referenzdaten verwendet werden. Mit anderen Worten bedeutet dies, dass die Referenzdaten aufgeschlüsselt nach bestimmten Einflussfaktoren, beispielsweise der Zahl der tatsächlich in der Infrastruktureinheit anwesenden Personen und/oder den Kalendertag, ermittelt werden, so dass für eine aktuelle Personenanzahl und/oder einen aktuellen Kalendertag die passenden Referenzdaten herangezogen werden können und diese mithin deutlich genauer und besser aufgeschlüsselt vorliegen, was die Verlässlichkeit der Aktivitätsüberwachung weiter erhöht. Bezüglich des Kalendertags kann beispielsweise unterschieden werden zwischen Werktagen, Wochenende, Feiertagen, unterschiedlichen Jahreszeiten und dergleichen.

Möglich und zweckmäßig ist ferner eine Ausgestaltung, in der bei Vorliegen von nach wenigstens einem Einflussfaktor aufgeschlüsselten Referenzdaten aktuell zu verwendende Referenzdaten auf der Grundlage einer wenigstens einen des wenigstens einen Einflussfaktors beschreibenden Benutzereingabe ausgewählt werden. Die Benutzereingabe kann beispielsweise von der wenigstens einen Person selbst an einer Eingabeeinrichtung erfolgen, beispielsweise kodiert nach bestimmten, umschriebenen Einflussfaktorsätzen. So kann eine Person beispielsweise Informationen wie "bin allein", "bin nicht im Haus", "habe Gäste im Haus", "liege heute im Bett" und dergleichen an die das Verfahren durchführende Vorrichtung vermitteln, welche aus den für wenigstens einen Einflussfaktor aufgeschlüsselten Referenzdaten dann die tatsächlich passenden Referenzdaten für den Vergleich auswählen kann. Es sei angemerkt, dass für mindestens einen Teil der verwendeten Einflussfaktoren, beispielsweise den Kalendertag oder sogar anwesende Personen, auch eine automatische Ermittlung vorgesehen sein kann und bevorzugt ist.

Der Vergleich kann jeweils für mehrere Zeitdauern und/oder wenigstens ein gleitendes Zeitfenster als Zeitdauer durchgeführt werden. Das bedeutet, zum einen ist es möglich, den Vergleich auf unterschiedlichen "Zeitskalen" vorzunehmen, um sowohl Langzeittrends als auch Kurzzeittrends feststellen zu können. Zweckmäßig ist es alternativ oder zusätzlich, ein gleitendes Zeitfenster als Zeitdauer zu verwenden, welches beispielsweise eine bestimmte Stundenzahl enthalten kann und ständig mitgeführt wird, um so möglichst schnell bei einer starken Veränderung der Verbrauchsaktivitäten einen kritischen Zustand erkennen zu können.

Wie bereits angedeutet wurde, kann es zudem zweckmäßig sein, wenn das Aktivitätskriterium benutzerseitig hinsichtlich der Erfolgsschwelle für den Vergleich anpassbar ist. Dies ist insbesondere dann zweckmäßig, wenn es möglich sein soll, die Analyse durch das Aktivitätskriterium scharf oder weniger scharf einzustellen. Auf diese Weise kann letztlich die Verlässlichkeit gegen das Risiko von Fehlauslösungen abgewogen werden.

Zweckmäßigerweise kann das Aktivitätskriterium zusätzlich Verbrauchswertverläufe für wenigstens ein weiteres Verbrauchsmedium, insbesondere Wärme und/oder Gas, auswerten. Vorteilhaft ist es mithin, zumindest dann, wenn weitere Verbrauchswertverläufe vorliegen, beispielsweise ein Wärmezähler und/oder ein Gaszähler als weitere Messeinrichtungen vorhanden sind, auf diese Informationen entsprechend für die Auswertung innerhalb des Aktivitätskriteriums heranzuziehen, da es sich auch hier übliche, insbesondere mit dem Strom- und Wasserverbrauch korrelierte, Verbrauchsmuster ergeben können, die angelernt werden können und zum Vergleich mit aktuellen Profildaten dienen können. Zusätzlich zu den Verbrauchswertverläufen von Wasser und Strom können die Profildaten dann auch Verbrauchswertverläufe für Wärme und/oder Gas (und/oder weitere, hier nicht genannte Verbrauchsmedien) enthalten. Auf diese Weise wird die Verlässlichkeit weiter erhöht.

Dabei sei an dieser Stelle noch angemerkt, dass die Leistungsfähigkeit des hier beschriebenen Aktivitätsmonitorings mit der Zahl der betrachteten Verbrauchsmedien skaliert. Würde nur eine Messgröße verwendet, beispielsweise nur Wasser als Verbrauchsmedium betrachtet, wäre eine Überwachung zwar mit sehr einfachen Mitteln bereits möglich, aber eher ungenau. Mehr überwachte Verbrauchswertverläufe erhöhen zwar den Aufwand, aber auch die Leistungsfähigkeit (weniger Zeitverzögerung, bis eine Nichtaktivität auffällt, weniger Fehlalarme). Denkbar ist daher eine Ausgestaltung der Erfindung auch derart, dass zu einem Basispaket, mit dem einen Überwachung von Verbrauchswertverläufen für Strom und Wasser möglich ist, modulare Erweiterungspakete (Software und/oder Hardware) vorgesehen werden, beispielsweise wenigstens für Gas und/oder Wärme. Dann existiert mithin die Möglichkeit, nachrüstbare Erweiterungen zur Erhöhung der Leistungsfähigkeit bereitzustellen.

Eine weitere Möglichkeit zur Erhöhung der Verlässlichkeit ist die Nutzung zusätzlicher Sensoren, was jedoch im Vergleich weniger bevorzugt ist, da diese grundsätzlich aufgrund des hier beschriebenen Aktivitätsmonitorings, zumindest im Dauerbetrieb, auch vermieden werden können.

Nichtsdestotrotz ist festzuhalten, dass sich Abweichungen von üblichem Verbrauchsverhalten typischerweise erst nach Ablauf einer bestimmten Zeitspanne hinreichend deutlich erkennen lassen, das bedeutet, die Erkennung von außergewöhnlichen Zuständen alleine auf Basis von Strom- und Wasserverbräuchen (und gegebenenfalls weiteren Verbräuchen) findet innerhalb einiger Stunden statt. Soll die Latenzzeit verbessert werden, ist es durchaus möglich, zusätzliche Sensoren einzusetzen.

Das erfindungsgemäße Verfahren sieht diesbezüglich vor, dass als Alarmmaßnahme wenigstens ein Aktivitätssensor aktiviert und dessen Sensordaten durch ein Notfallkriterium ausgewertet werden, bei dessen Erfüllung eine Notfallmaßnahme, insbesondere die Kontaktierung eines Notfalldienstes und/oder einer Kontaktperson, eingeleitet wird. Mithin können, um Aufwand zu sparen, zusätzliche Sensoren durchaus vorgesehen werden, müssen jedoch nicht dauerhaft betrieben werden, sondern können bei Fehlschlagen des Aktivitätskriteriums insbesondere erst in Betrieb genommen werden. Lässt sich durch diese Sensoren ein eindeutiger Notfall erkennen, kann eine Notfallmaßnahme eingeleitet werden, beispielsweise ein Notfalldienst, insbesondere ein ärztlicher Notfalldienst, benachrichtigt werden und/oder eine sonstige Kontaktperson kontaktiert werden.

Beispiele für solche Aktivitätssensoren, die im Rahmen der vorliegenden Erfindung eingesetzt werden können, umfassen eine auf einem Fußboden der Infrastruktureinheit liegende Person detektierende Fußbodensensoren und/oder Infrarotsensoren zur Sturzerkennung und/oder Bewegungsmelder und/oder Infrarotsensoren in Heizkostenverteilern und/oder die Atemluft in der Infrastruktureinheit, insbesondere den CO₂-Gehalt, vermessende Atemluftsensoren und/oder einzelnen Verbrauchern zugeordnete Stromsensoren. Die Verwendung derartiger Aktivitätssensoren ist im Stand der Technik bereits weitgehend bekannt und muss hier nicht genauer dargelegt werden. Bevorzugt werden als zusätzliche Aktivitätssensoren jedoch solche verwendet, die üblicherweise ohnehin vorgesehen sind oder vorgesehen sein sollten, was Infrastruktureinheiten angeht. So ist beispielsweise vorgeschlagen worden, zukünftig in Infrastruktureinheiten, insbesondere Wohnungen und/oder Häusern, Atemluftsensoren, die den CO₂-Gehalt messen, ohnehin vorzusehen.

Vorzugsweise kann als Alarmmaßnahme über eine Ausgabeeinrichtung, insbesondere eine der Infrastruktureinheit und/oder eine der Person zugeordnete Ausgabeeinrichtung, eine Aufforderung für eine Bedieneingabe an die Person ausgegeben werden, wobei dann, wenn die Person innerhalb einer Bedienzeitspanne keine Bedieneingabe durchführt, eine Notfallmaßnahme, insbesondere die Kontaktierung eines Notfalldienstes und/oder einer Kontaktperson, eingeleitet wird. Um mithin Fehlalarme zu vermeiden, ist es denkbar, erst dann weitere Personen zu informieren, wenn innerhalb einer definierten Zeitspanne die wenigstens eine Person nicht ein Bedienelement betätigt hat, welches anzeigt, dass kein kritischer Zustand vorliegt. Eine solche Aufforderung kann beispielsweise über eine Ausgabeeinrichtung der Infrastruktureinheit, beispielsweise einen Computer, einen Fernseher oder dergleichen, ausgegeben werden, oder auch auf einer der Person zugeordneten Ausgabeeinrichtung, beispielsweise einem Mobiltelefon, erfolgen. Entsprechende Bedienelemente können ebenso an speziell vorgesehenen Bedieneinrichtungen oder aber auch an der entsprechenden Ausgabeeinrichtung selber erfolgen. Insbesondere im Kontext mit einer anpassbaren Erfolgsschwelle für den Vergleich des Aktivitätskriteriums kann so beispielsweise ein Auslösen schärfer gestellt werden oder aber es ist möglich, die Zeitdauern und somit die Reaktionszeit zu verkürzen, mit dem Risiko, dass häufiger Aufforderungen ergehen, das Bedienelement als "alles in Ordnung"-Taste zu betätigen.

Selbstverständlich kann auch grundsätzlich im Rahmen der vorliegenden Erfindung vorgesehen sein, dass als Alarmmaßnahme eine Alarminformation an eine Kontaktperson und/oder einen Notfalldienst gesendet wird. Beispielsweise kann eine mit der wenigstens einen zu überwachenden Person verwandte Person benachrichtigt werden und/oder ein professioneller Dienstleiter kann aktiv werden.

Neben dem Verfahren betrifft die vorliegende Erfindung auch eine entsprechende Vorrichtung zur Aktivitätsüberwachung wenigstens einer Person in einer Infrastruktureinheit, die mit Verbrauchsmedien, umfassend wenigstens Strom und Wasser, versorgt wird, wobei jedem Verbrauchsmedium eine Messeinrichtung zur Messung eines Verbrauchswerts des Verbrauchsmediums zugeordnet ist, welche aufweist:
- eine Datenschnittstelle zur Entgegennahme von zeitliche Verbrauchswertverläufe wenigstens für Strom und Wasser für eine vorbestimmte Zeitdauer enthaltenden Profildaten, die durch die Messeinrichtungen bereitgestellt werden,
- eine Speichereinrichtung zur Speicherung von ein eine normale Aktivität beschreibendes Referenzprofil beschreibenden Referenzdaten, und
- eine Auswerteeinrichtung zur Auswertung der Profildaten zur Bestimmung der Erfüllung eines Aktivitätskriteriums mittels eines Vergleichs der Profildaten mit ein Vergleichsprofil als Teil des Referenzprofils für die Zeitdauer beschreibenden Referenzdaten, um festzustellen, ob und in welchem Maße Abweichungen von der normalen Aktivität vorliegen, und zur Auslösung wenigstens einer Alarmmaßnahme, wenn das Aktivitätskriterium bei einem fehlgeschlagenen Vergleich nicht erfüllt ist, also keine normale Aktivität anzeigt, wobei die Referenzdaten Kreuzkorrelationen zwischen dem Wasserverbrauch und dem Stromverbrauch beschreiben und wobei als Alarmmaßnahme die Aktivierung wenigstens eines Aktivitätssensors und eine Auswertung von dessen Sensordaten durch ein Notfallkriterium erfolgt, wobei bei dessen Erfüllung die Einleitung einer Notfallmaßnahme erfolgt.

Sämtliche Ausführungen zum erfindungsgemäßen Verfahren lassen sich entsprechend auf die erfindungsgemäße Vorrichtung übertragen, so dass auch mit dieser die bereits genannten Vorteile erhalten werden können. Dabei können verschiedene Datenschnittstellen eingesetzt werden, um die Verbrauchswerte bzw. Verbrauchswertverläufe von den Messeinrichtungen zu erhalten. Beispielsweise können lokale, drahtlose Schnittstellen wie Bluetooth oder Wireless-M-Bus oder dergleichen verwendet werden, wenn die Vorrichtung zumindest teilweise lokal installiert ist; selbstverständlich sind auch drahtgebundene Schnittstellen zumindest teilweise denkbar, insbesondere dann, wenn die Vorrichtung als Teil einer Messeinrichtung realisiert wird und/oder wenigstens teilweise eine drahtgebundene Verbindung genutzt werden soll, beispielsweise bei einer entfernt angeordneten, insbesondere für mehrere Infrastruktureinheiten zuständigen Vorrichtung, beispielsweise einem Server, der die Verbrauchswerte wenigstens teilweise über das Internet erhält. Ersichtlich sind viele verschiedene Ausgestaltungen der Vorrichtung denkbar, wobei es bevorzugt ist, eine zentrale, Personen in verschiedenen Infrastruktureinheiten überwachende Vorrichtung vorzusehen.

Denkbar ist es jedoch auch, insbesondere bei der zunehmenden Intelligenz in Messeinrichtungen (Stichwort "smart meter"), die erfindungsgemäße Funktionalität in einer Messeinrichtung zu realisieren. Eine erfindungsgemäße Messeinrichtung für Strom oder Wasser als einer Infrastruktureinheit zuzuführendem Verbrauchsmedium weist mithin eine erfindungsgemäße Vorrichtung auf. Auch diesbezüglich gelten selbstverständlich die Ausführungen zum Verfahren und zur Vorrichtung fort.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: ein System zur Durchführung des erfindungsgemäßen Verfahrens,
- Fig. 2: einen Ablaufplan eines Ausführungsbeispiels des erfindungsgemäßen Verfahrens, und
- Fig. 3: einen beispielhaften Verbrauchswertverlauf.

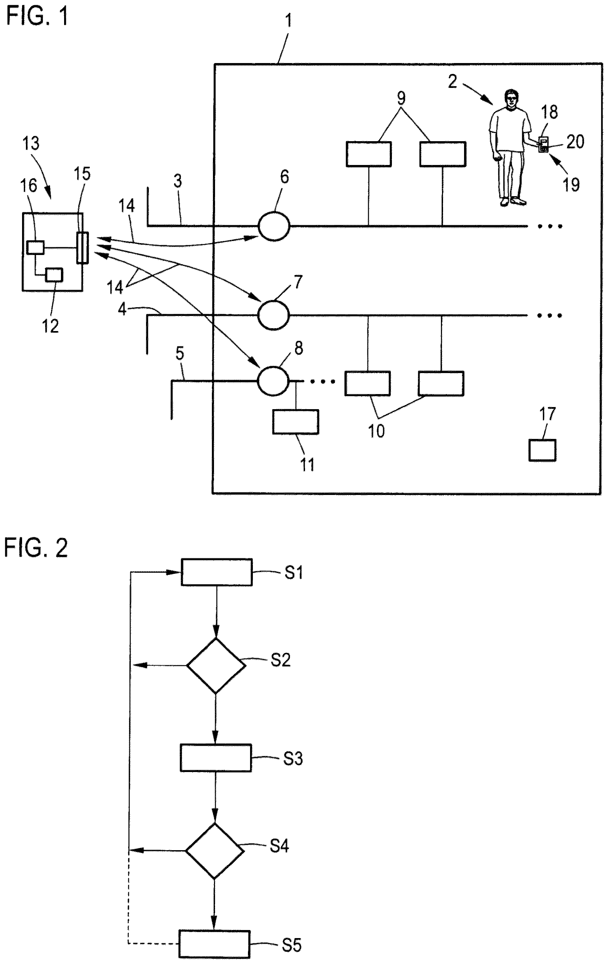

Fig. 1 zeigt ein System, in dem das erfindungsgemäße Verfahren durchgeführt werden kann. Angedeutet ist eine Infrastruktureinheit 1, bei der es sich vorliegend um eine Wohnung oder ein Haus handeln kann. Die Infrastruktureinheit 1 wird bewohnt von wenigstens einer bezüglich ihrer Aktivität zu überwachenden Person 2, um kritische, außergewöhnliche Zustände der Person 2 feststellen zu können. Um die Infrastruktureinheit 1 mit Verbrauchsmedien versorgen zu können, sind verschiedene Zuleitungen 3, 4, 5 vorhanden, vorliegend beispielhaft eine Zuleitung 3 für Wasser, eine Zuleitung 4 für Strom und eine Zuleitung 5 für Gas. Jeder der Zuleitungen 3, 4, 5 ist jeweils eine Messeinrichtung 6, 7, 8 zur Erfassung des Verbrauchs des jeweiligen Verbrauchsmediums zugeordnet. Mithin handelt es sich bei der Messeinrichtung 6 um einen Wasserzähler, bei der Messeinrichtung 7 um einen Stromzähler und bei der Messeinrichtung 8 um einen Gaszähler. Die Messeinrichtungen 6, 7, 8 können vorliegend sowohl einen akkumulierten Verbrauchswert als auch einen aktuellen, auf eine Zeiteinheit bezogenen Verbrauchswert ermitteln.

Die Verbrauchsmedien werden in entsprechenden, von der Person 2 verwendeten Verbrauchern 9, 10 und 11 genutzt, von denen hier nur beispielhaft einige elektrische Verbraucher 10, Wasserverbraucher 9 und Gasverbraucher 11 angezeigt sind. Die Stromverbraucher 10 können beispielsweise einen Herd, einen Backofen, eine Mikrowelle, eine Spülmaschine, eine Waschmaschine, einen Gefrierschrank, einen Kühlschrank, ein Fernsehgerät, ein Radio, einen Computer und/oder Lichtquellen umfassen. Die Wasserverbraucher 9 wiederum umfassen beispielsweise eine Dusche, eine Toilette, ein Handwaschbecken, eine Badewanne, ein Spülbecken, eine Spülmaschine, eine Waschmaschine und einen Wasserhahn für die Gartenbewässerung. Die Gasverbraucher 11 umfassen beispielsweise einen Herd, eine Gastherme für eine Heizung und eine Gastherme für warmes Wasser.

Der Verbraucher 2 legt in der Infrastruktureinheit 1 dabei ein typisches Nutzungsverhalten an den Tag, das in seinen wiederkehrenden Mustern durch ein Referenzprofil beschrieben werden kann. Ein derartiges Referenzprofil kann durch Auswertung von Profildaten über einen längeren Zeitraum als Trainingsdaten ermittelt werden, wobei beispielsweise Verfahren des maschinellen Lernens, insbesondere der künstlichen Intelligenz und/oder der statistischen Analyse, eingesetzt werden können. Die Referenzdaten, die das Referenzprofil beschreiben, werden dabei aufgeschlüsselt nach der Personenanzahl in der Infrastruktureinheit 1 (falls anwendbar) und nach Kalendertagen ermittelt, wobei jeweils ein ganzer Tag abgebildet wird, so dass für vorgegebene Zeitdauern Vergleichsprofile aus den Referenzprofilen extrahiert werden können.

Aktuelle Referenzdaten, die das aktuelle Referenzprofil der Person 2 beschreiben, liegen in einer Speichereinrichtung 12 einer erfindungsgemäßen Vorrichtung 13 vor, die vorliegend extern zu der Infrastruktureinheit 1 gezeigt ist, aber in anderen Ausführungsbeispielen auch innerhalb der Infrastruktureinheit 1 vorliegen kann, insbesondere sogar als Teil einer der Messeinrichtungen 6 oder 7. Im Ausführungsbeispiel nach Fig. 1 ist die Vorrichtung 13 als ein zentraler Server ausgebildet, auf dem eine Aktivitätsüberwachung für mehrere Infrastruktureinheiten 1 und entsprechende Personen 2 erfolgen kann. Um dies zu ermöglichen, ist die Vorrichtung 13 über Kommunikationsverbindungen 14, die auch das Internet umfassen, mit den Messeinrichtungen 6, 7 und 8 verbunden. Über eine Datenschnittstelle 15 können entsprechende Verbrauchswerte entgegengenommen werden, so dass Verbrauchswertverläufe über Zeitdauern für zumindest Wasser und Strom entstehen. Zweckmäßig und vorliegend vorgesehen ist es jedoch auch, weitere Verbrauchswertverläufe, beispielsweise bezüglich des Gasverbrauchs (Messeinrichtung 8), sowohl in den Referenzdaten als auch bei der Aktivitätsüberwachung zu berücksichtigen.

Die entstehenden Verbrauchswertverläufe werden als Profildaten einer Auswerteeinheit 16 der Vorrichtung 13 zugeführt, welche die Profildaten durch ein Aktivitätskriterium auswertet, das letztlich die aktuellen Verbrauchswertverläufe der Profildaten mit einem für die Zeitdauer aus den Referenzdaten extrahierten Vergleichsprofil, also einem ausgewählten Teil der Referenzdaten, vergleicht, worauf im Folgenden noch genauer eingegangen werden wird. Schlägt dieser Vergleich fehl, ist von einer außergewöhnlichen Situation auszugehen und es werden Alarmmaßnahmen durchgeführt, wobei eine dieser Alarmmaßnahmen vorliegend auch eine der Person 2 zugeordnete Ausgabeeinrichtung 18 in Form eines Mobiltelefons 19 nutzt, wobei die Person 2 aufgefordert wird, eine Bedieneingabe mittels Bedienelementen 20 vorzunehmen, um anzuzeigen, dass dennoch alles in Ordnung ist.

Es sei noch angemerkt, dass in der Infrastruktureinheit 1 auch Aktivitätssensoren 17 vorliegen, vorzugsweise ohnehin verbaute Aktivitätssensoren 17, beispielsweise ein Atemluftsensor, der den CO₂-Gehalt der Luft in verschiedenen Räumen der Infrastruktureinheit 1 misst. Sensordaten solcher Aktivitätssensoren 17 werden subsidiär im Sinne einer Alarmmaßnahme genutzt.

Fig. 2 zeigt einen Ablaufplan eines Ausführungsbeispiels des erfindungsgemäßen Verfahrens, wie es durch die Vorrichtung 13 durchgeführt werden kann. Dabei werden in einem Schritt S1 Verbrauchswertverläufe zumindest für Wasser und Strom mittels der Messeinrichtungen 6, 7 und gegebenenfalls auch für Gas mittels der Messeinrichtung 8 erfasst. Diese Verbrauchswertverläufe werden im Schritt S2 durch ein Aktivitätskriterium ausgewertet. Die Verbrauchswertverläufe beziehen sich auf wenigstens eine Zeitdauer. Für diese Zeitdauer, beispielsweise ein mehrere Stunden umfassendes Intervall eines Tages, wird ein der Zeitdauer entsprechender Teil der Referenzdaten, die mithin ein Vergleichsprofil wiedergeben, ausgewählt. Das Vergleichsprofil beschreibt eine normale Aktivität der Person 2 in der Infrastruktureinheit 1. Dabei sei darauf hingewiesen, dass es sich bei den Referenzdaten des Vergleichsprofils nicht zwangsläufig ebenso um Verbrauchswertverläufe handeln muss, sondern die Referenzdaten auch deren charakteristische Muster und Zusammenhänge beschreiben, vorliegend zumindest Kreuzkorrelationen zwischen dem Wasserverbrauch und dem Stromverbrauch (sowie gegebenenfalls dem Gasverbrauch). Wenigstens eine der wenigstens einen Zeitdauer ist hier dabei als ein gleitendes Zeitfenster ausgebildet.

Im Rahmen des Aktivitätskriteriums im Schritt S2 werden nun die Profildaten für die Zeitdauer mit den das Vergleichsprofil beschreibenden Referenzdaten für die Zeitdauer verglichen, indem ein Korrelationswert zwischen den Profildaten und den das Vergleichsprofil beschreibenden Referenzdaten ermittelt wird. Der Korrelationswert gibt dabei an, inwieweit die in den Profildaten enthaltenen Muster und Zusammenhänge den Mustern und Zusammenhängen in den das Vergleichsprofil beschreibenden Referenzdaten entsprechen. Mit anderen Worten handelt es sich bei dem Korrelationswert um ein Übereinstimmungsmaß im Hinblick auf Muster und Zusammenhänge.

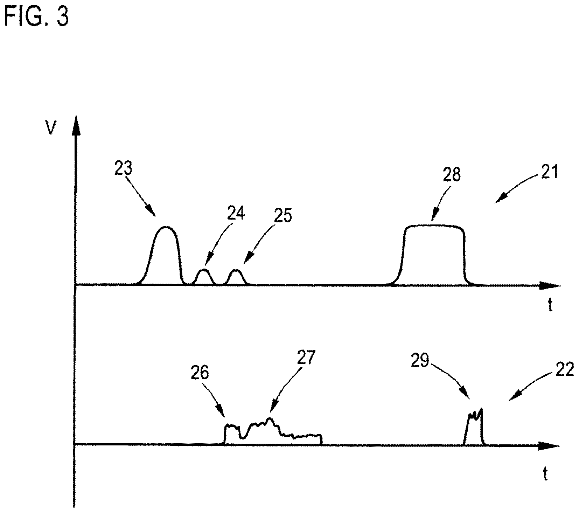

Dies sei im Hinblick auf die beispielhaften Verbrauchswertverläufe 21, 22 der Fig. 3 näher erläutert. Es ist dabei anzumerken, dass die Verbrauchswertverläufe 21, 22 stark vereinfacht und illustrierend dargestellt sind und nicht zwangsläufig echten Verbrauchswertverläufen entsprechen müssen. Der Verbrauchswertverlauf 21 ist dabei für Wasser, der Verbrauchswertverlauf 22 für Strom vorgesehen. Gezeigt ist eine Situation am Morgen. Die Person 2 steht dann üblicherweise auf und benutzt die Toilette, wobei der durch die Wasserspülung entstehende Wasserverbrauch durch den Peak 23 symbolisiert ist, das folgende Händewaschen durch den Peak 24. Die Person 2 geht sodann in die Küche der Infrastruktureinheit 1, um dort, Peak 25, Wasser abzufüllen und, Peak 26 des Strom-Verbrauchswertverlaufs 22, eine Kaffeemaschine oder einen Wasserkocher in Betrieb zu nehmen. Der weitere Verlauf 22 der Strom-Verbrauchswerte ist durch die Nutzung verschiedener elektrischer Verbraucher 9 der Küche gekennzeichnet, Bereich 27. Nach dem Frühstücken verlässt die Person 2 die Küche der Infrastruktureinheit 1 und begibt sich wieder in das Bad, um zu duschen, Peak 28 des Wasser-Verbrauchwerteverlaufs 21. Föhnt sich die Personen 2 sodann, ist dies wiederum an einem Peak 29 im Strom-Verbrauchswerteverlauf 22 zu erkennen.

Es wird deutlich, dass im normalen Tagesablauf der Person 2 eine Vielzahl von wiederkehrenden und/oder miteinander korrelierenden Ereignissen auftreten, wobei die Korrelation sich durchaus auch auf Zusammenhänge zwischen den Verbrauchswertverläufen 21, 22 für Wasser und Strom beziehen, was den Ablauf oder zeitliche Koinzidenzen, beispielsweise hinsichtlich einer Kaffeemaschine oder eines Wasserkochers bzw. bei der Nutzung von Waschmaschinen und Geschirrspülmaschinen, ergibt. Mithin ist es auch die Zusammenschau von Verbrauchswertverläufen 21, 22 wenigstens für Wasser und Strom, die dem hier beschriebenen Vorgehen seine Verlässlichkeit und Robustheit verleiht. Denn beispielsweise durch "smart data analytics" lassen sich unter Verwendung maschinellen Lernens diese Muster und Zusammenhänge erkennen, extrahieren und in den Referenzdaten als Referenzprofil zusammenfassen, unterscheidend zwischen Kalendertagen (Jahreszeiten, Feiertage, Wochenende, Werktage, ...) und auch der Zahl tatsächlich anwesender Personen 2.

Gerade dann, wenn nach Einflussfaktoren (beispielsweise umfassend Kalendertag, Anzahl Personen, Grundaktivitätsinformationen) aufgeschlüsselte Referenzdaten vorliegen, kann im übrigen, falls nicht alle Einflussfaktoren ohnehin automatisch ermitteltbar sind, dem Schritt S1 vorgeschaltet auch ein Schritt S0 denkbar sein, in dem eine Benutzereingabe, die wenigstens einen Einflussfaktor beschreibend, entgegengenommen wird, welche im Schritt S2 bei der Auswahl der passenden Referenzdaten berücksichtigt werden kann.

Im Schritt S2 gilt das Aktivitätskriterium als erfüllt, wenn der Korrelationswert einen Schwellwert übersteigt. Dieser Schwellwert kann, wie gegebenenfalls auch die Länge der wenigstens einen betrachteten Zeitdauer, benutzerseitig anpassbar sein, um einzustellen, wie leicht oder schwer das Aktivitätskriterium "auslöst". Ist das Aktivitätskriterium erfüllt, wird von einer normalen Aktivität der Person 2 ausgegangen und es werden für den nächsten zyklischen Überwachungsschritt wieder in Schritt S1 neue Profildaten ermittelt.

Schlägt das Aktivitätskriterium, genauer gesagt der Vergleich, jedoch fehl, wird in einem Schritt S3 fortgefahren. Dort wird wenigstens eine Alarmmaßnahme durchgeführt, wobei vorliegend zunächst eine optionale Alarmmaßnahme betrachtet werden soll, bei der eine Aufforderung zu einer Bedieneingabe über die Ausgabeeinrichtung 18 der Person 2 ausgegeben wird. Dabei sei darauf hingewiesen, dass alternativ oder zusätzlich auch feststehende, der Infrastruktureinheit 1 zugeordnete Ausgabeeinrichtungen verwendet werden können. Dann wird in einem Schritt S4 für eine vorbestimmte Zeitspanne gewartet. Tritt in dieser vorbestimmten Zeitspanne die Bedieneingabe auf, bestätigt also die Person 2, dass alles in Ordnung ist, wird wieder mit Schritt S1 fortgefahren. Erfolgt jedoch keine Bestätigung der Person 2, dass alles in Ordnung ist, wird von einem Notfall ausgegangen und in Schritt S5 wird wenigstens eine Notfallmaßnahme ausgeführt. Eine solche Notfallmaßnahme kann insbesondere die Kontaktierung eines Notfalldienstes, beispielsweise eines Rettungsdienstes und/oder sonstigen professionellen Dienstleisters, umfassen, und/oder auch die Kontaktierung einer Kontaktperson, beispielsweise eines Verwandten.

Es sei noch darauf hingewiesen, dass selbstverständlich auch andere Alarmmaßnahmen zusätzlich oder alternativ vorgesehen sein können, beispielsweise bereits dort die Kontaktierung einer Kontaktperson und/oder eines Notfalldienstes. Erfindungsgemäß erfolgt im Schritt S3 als Alarmmaßnahme die Aktivierung von Aktivitätssensoren 17 bzw. Auswertung von Sensordaten von Aktivitätssensoren 17 durch ein Notfallkriterium, bei dessen Erfüllung eine Notfallmaßnahme eingeleitet wird.

Es sei schließlich als weiteres Beispiel noch angemerkt, dass sich viele wiederkehrende Muster in einem Haushalt, was Verbräuche angeht, auch mit einem Kühlschrank in Zusammenhang setzen lassen. Ein Kühlschrank stellt einen Mittelpunkt des häuslichen Lebens dar. Mithin kann eine Erweiterung der Erfindung auch vorsehen, dass als zusätzlicher Sensorwert mit einem dedizierten Sensor ein Verbrauch einer Innenbeleuchtungseinrichtung des Kühlschranks vermessen wird, woraus sich weitere zur Verfeinerung des Aktivitätsmonitorings geeignete Muster ableiten lassen.

## Patentansprüche

1. Verfahren zur Aktivitätsüberwachung wenigstens einer Person (2) in einer Infrastruktureinheit (1), die mit Verbrauchsmedien, umfassend wenigstens Strom und Wasser, versorgt wird, wobei jedem Verbrauchsmedium eine Messeinrichtung (6, 7, 8) zur Messung eines Verbrauchswerts des Verbrauchsmediums zugeordnet ist, wobei durch die Messeinrichtungen (6, 7, 8) wenigstens für Strom und Wasser zeitliche Verbrauchswertverläufe (21, 22) für eine vorbestimmte Zeitdauer als Profildaten bereitgestellt werden (S1) und in einer Speichereinrichtung (12) der das Verfahren durchführenden Vorrichtung (13) Referenzdaten, die ein eine normale Aktivität beschreibendes Referenzprofil beschreiben, vorliegen, wobei zur Bestimmung der Erfüllung eines Aktivitätskriteriums ein Vergleichsprofil für die Zeitdauer beschreibende Referenzdaten mit den Profildaten verglichen werden, um festzustellen, ob und in welchem Maße Abweichungen von der normalen Aktivität vorliegen (S2), wobei, wenn das Aktivitätskriterium bei einem fehlgeschlagenen Vergleich nicht erfüllt ist, also keine normale Aktivität anzeigt, wenigstens eine Alarmmaßnahme durchgeführt wird (S3), wobei die Referenzdaten Kreuzkorrelationen zwischen dem Wasserverbrauch und dem Stromverbrauch beschreiben und als Alarmmaßnahme wenigstens ein Aktivitätssensor (17) aktiviert und dessen Sensordaten durch ein Notfallkriterium ausgewertet werden, bei dessen Erfüllung eine Notfallmaßnahme eingeleitet wird.

2. Verfahren nach Anspruch 1, **dadurch gekennzeichnet, dass** im Rahmen des Vergleichs ein die Übereinstimmung mit dem Vergleichsprofil beschreibender Korrelationswert ermittelt und mit einem Schwellwert verglichen wird.

3. Verfahren nach Anspruch 1 oder 2, **dadurch gekennzeichnet, dass** personenspezifische Referenzdaten ermittelt werden, insbesondere in einer Lernphase durch Auswertung von Profildaten der Person (2) als Trainingsdaten in der Lernphase mittels eines Lernalgorithmus der künstlichen Intelligenz und/oder der statistischen Datenauswertung.

4. Verfahren nach Anspruch 3, **dadurch gekennzeichnet, dass** bei erfülltem Aktivitätskriterium die Referenzdaten unter Nutzung der der Erfüllung zugrunde liegenden Profildaten aktualisiert werden.

5. Verfahren nach Anspruch 3 oder 4, **dadurch gekennzeichnet, dass** die Referenzdaten abhängig von der Personenzahl in der Infrastruktureinheit (1) und/oder von dem Kalendertag ermittelt werden, wobei für den Vergleich im Aktivitätskriterium auf die aktuelle Personenzahl und/oder den aktuellen Kalendertag bezogene Referenzdaten verwendet werden.

6. Verfahren nach einem der vorangehenden Ansprüche, **dadurch gekennzeichnet, dass** der Vergleich für mehrere Zeitdauern und/oder für wenigstens ein gleitendes Zeitfenster als Zeitdauer durchgeführt wird und/oder das Aktivitätskriterium benutzerseitig hinsichtlich der Erfolgsschwelle für den Vergleich anpassbar ist.

7. Verfahren nach einem der vorangehenden Ansprüche, **dadurch gekennzeichnet, dass** das Aktivitätskriterium zusätzlich Verbrauchswertverläufe (21, 22) für wenigstens ein weiteres Verbrauchsmedium, insbesondere Wärme und/oder Gas, auswertet.

8. Verfahren nach einem der vorangehenden Ansprüche, **dadurch gekennzeichnet, dass** als Aktivitätssensoren (17) eine auf einem Fußboden der Infrastruktureinheit (1) liegende Person (2) detektierende Fußbodensensoren und/oder Infrarotsensoren zur Sturzerkennung und/oder Bewegungsmelder und/oder Infrarotsensoren in Heizkostenverteilern und/oder die Atemluft in der Infrastruktureinheit (1) insbesondere den CO2-Gehalt, vermessende Atemluftsensoren und/oder einzelnen Verbrauchern zugeordnete Stromsensoren verwendet werden.

9. Verfahren nach einem der vorangehenden Ansprüche, **dadurch gekennzeichnet, dass** als Alarmmaßnahme über eine Ausgabeeinrichtung (18), insbesondere eine der Infrastruktureinheit (1) und/oder eine der Person (2) zugeordnete Ausgabeeinrichtung (18), eine Aufforderung für eine Bedieneingabe an die Person (2) ausgegeben wird, wobei dann, wenn die Person (2) innerhalb einer Bedienzeitspanne keine Bedieneingabe durchführt, eine Notfallmaßnahme, insbesondere die Kontaktierung eines Notfalldienstes und/oder einer Kontaktperson, eingeleitet wird.

10. Verfahren nach einem der vorangehenden Ansprüche, **dadurch gekennzeichnet, dass** als Alarmmaßnahme eine Alarminformation an eine Kontaktperson und/oder einen Notfalldienst gesendet wird.

11. Vorrichtung (13) zur Aktivitätsüberwachung wenigstens einer Person (2) in einer Infrastruktureinheit (1), die mit Verbrauchsmedien, umfassend wenigstens Strom und Wasser, versorgt wird, wobei jedem Verbrauchsmedium eine Messeinrichtung (6, 7, 8) zur Messung eines Verbrauchswerts des Verbrauchsmediums zugeordnet ist, aufweisend:
- eine Datenschnittstelle (15) zur Entgegennahme von zeitliche Verbrauchswertverläufe (21, 22) wenigstens für Strom und Wasser für eine vorbestimmte Zeitdauer enthaltenden Profildaten, die durch die Messeinrichtungen (6, 7, 8) bereitgestellt werden,
- eine Speichereinrichtung (12) zur Speicherung von ein eine normale Aktivität beschreibendes Referenzprofil beschreibenden Referenzdaten und
- eine Auswerteeinrichtung (16) zur Auswertung der Profildaten zur Bestimmung der Erfüllung eines Aktivitätskriteriums mittels eines Vergleichs der Profildaten mit ein Vergleichsprofil als Teil des Referenzprofils für die Zeitdauer beschreibenden Referenzdaten, um festzustellen, ob und in welchem Maße Abweichungen von der normalen Aktivität vorliegen, und zur Auslösung wenigstens einer Alarmmaßnahme, wenn das Aktivitätskriterium bei einem fehlgeschlagenen Vergleich nicht erfüllt ist, also keine normale Aktivität anzeigt, wobei die Referenzdaten Kreuzkorrelationen zwischen dem Wasserverbrauch und dem Stromverbrauch beschreiben und wobei als Alarmmaßnahme die Aktivierung wenigstens eines Aktivitätssensors (17) und eine Auswertung von dessen Sensordaten durch ein Notfallkriterium erfolgt, wobei bei dessen Erfüllung die Einleitung einer Notfallmaßnahme erfolgt.

12. Messeinrichtung (6, 7, 8) für Strom oder Wasser als einer Infrastruktureinheit (1) zuzuführendem Verbrauchsmedium, aufweisend eine Vorrichtung (13) nach Anspruch 11.

## Claims

1. Method for monitoring the activity of at least one person (2) in an infrastructure unit (1) supplied with consumption media comprising at least power and water, wherein a measuring device (6, 7, 8) for measuring a consumption value of the consumption medium is assigned to each consumption medium, wherein temporal consumption value profiles (21, 22) are provided (S1), at least for power and water, for a predetermined period of time as profile data by the measuring devices (6, 7, 8), and reference data which describe a reference profile describing a normal activity are available in a storage device (12) of the apparatus (13) carrying out the method, wherein, in order to determine whether an activity criterion is complied with, a comparison profile for reference data describing the period of time are compared with the profile data in order to determine whether and the extent to which there are deviations from the normal activity (S2), wherein, if the activity criterion is not complied with in the event of a failed comparison, that is to say does not indicate any normal activity, at least one alarm measure is carried out (S3), wherein the reference data describe cross-correlations between the water consumption and the power consumption, and, as an alarm measure, at least one activity sensor (17) is activated and its sensor data are evaluated by means of an emergency criterion which, if complied with, initiates an emergency measure.

2. Method according to Claim 1, **characterized in that** a correlation value describing the correspondence to the comparison profile is determined during the comparison and is compared with a threshold value.

3. Method according to Claim 1 or 2, **characterized in that** person-specific reference data are determined, in particular in a learning phase, by evaluating profile data relating to the person (2) as training data in the learning phase using an artificial intelligence and/or statistical data evaluation learning algorithm.

4. Method according to Claim 3, **characterized in that**, if the activity criterion is complied with, the reference data are updated using the profile data on which the compliance is based.

5. Method according to Claim 3 or 4, **characterized in that** the reference data are determined on the basis of the number of people in the infrastructure unit (1) and/or the calendar day, wherein reference data based on the current number of people and/or the current calendar day are used for the comparison in the activity criterion.

6. Method according to one of the preceding claims, **characterized in that** the comparison is carried out for a plurality of periods of time and/or for at least one sliding time window as a period of time, and/or the activity criterion can be adapted by the user with regard to the success threshold for the comparison.

7. Method according to one of the preceding claims, **characterized in that** the activity criterion additionally evaluates consumption value profiles (21, 22) for at least one further consumption medium, in particular heat and/or gas.

8. Method according to one of the preceding claims, **characterized in that** floor sensors detecting a person (2) lying on a floor of the infrastructure unit (1) and/or infrared sensors for detecting a fall and/or motion detectors and/or infrared sensors in heat cost allocators and/or respiratory air sensors measuring the respiratory air in the infrastructure unit (1), in particular the CO2 content, and/or current sensors assigned to individual consumers are used as activity sensors (17).

9. Method according to one of the preceding claims, **characterized in that** a request for an operating input is output to the person (2) as an alarm measure via an output device (18), in particular an output device (18) assigned to the infrastructure unit (1) and/or to the person (2), wherein an emergency measure, in particular contacting an emergency service and/or a contact person, is initiated when the person (2) does not make any operating input within an operating period of time.

10. Method according to one of the preceding claims, **characterized in that** alarm information is sent to a contact person and/or an emergency service as an alarm measure.

11. Apparatus (13) for monitoring the activity of at least one person (2) in an infrastructure unit (1) supplied with consumption media comprising at least power and water, wherein a measuring device (6, 7, 8) for measuring a consumption value of the consumption medium is assigned to each consumption medium, having:
- a data interface (15) for receiving profile data which contain temporal consumption value profiles (21, 22) at least for power and water for a predetermined period of time and are provided by the measuring devices (6, 7, 8),
- a storage device (12) for storing reference data which describe a reference profile describing a normal activity, and
- an evaluation device (16) for evaluating the profile data in order to determine whether an activity criterion is complied with by comparing the profile data with reference data describing a comparison profile as part of the reference profile for the period of time in order to determine whether and the extent to which there are deviations from the normal activity, and for initiating at least one alarm measure if the activity criterion is not complied with in the event of a failed comparison, that is to say does not indicate any normal activity, wherein the reference data describe cross-correlations between the water consumption and the power consumption, and wherein the activation of at least one activity sensor (17) and an evaluation of its sensor data by means of an emergency criterion are carried out as an alarm measure, wherein an emergency measure is initiated if the criterion is complied with.

12. Measuring device (6, 7, 8) for power or water as a consumption medium to be supplied to an infrastructure unit (1), having an apparatus (13) according to Claim 11.

## Revendications

1. Procédé de surveillance de l'activité d'au moins une personne (2) dans une unité d'infrastructure (1) qui est alimentée en milieux de consommation comprenant au moins l'électricité et l'eau, chaque milieu de consommation étant associé à un module de mesure (6, 7, 8) destiné à mesurer une valeur de consommation du milieu de consommation, les modules de mesure (6, 7, 8) fournissant des variations de valeurs de consommation dans le temps (21, 22) pour une durée prédéterminée comme données de profil au moins pour l'électricité et l'eau (S1), et des données de référence, qui décrivent un profil de référence décrivant une activité normale, étant présentes dans un dispositif de stockage (12) du dispositif (13) mettant en œuvre le procédé, pour déterminer si un critère d'activité est satisfait, des données de référence décrivant un profil de comparaison pour la durée étant comparées aux données de profil afin de déterminer si et dans quelle mesure des écarts existent par rapport à l'activité normale (S2),
si le critère d'activité n'est pas satisfait en cas d'échec de la comparaison, c'est-à-dire s'il n'indique pas une activité normale, au moins une mesure d'alarme étant réalisée (S3),
les données de référence décrivant des corrélations croisées entre la consommation d'eau et la consommation d'électricité et au moins un capteur d'activité (17) étant activé comme mesure d'alarme et les données de capteur étant évaluées par un critère d'urgence dont la satisfaction déclenche une mesure d'urgence.

2. Procédé selon la revendication 1, **caractérisé en ce que**, dans le cadre de la comparaison, une valeur de corrélation qui décrit la coïncidence avec le profil de comparaison étant déterminée et comparée à une valeur seuil.

3. Procédé selon la revendication 1 ou 2, **caractérisé en ce que** des données de référence spécifiques à une personne sont déterminées, en particulier dans une phase d'apprentissage, par évaluation de données de profil de la personne (2) comme données d'entraînement lors de la phase d'apprentissage à l'aide d'un algorithme d'apprentissage d'intelligence artificielle et/ou d'évaluation de données statistiques.

4. Procédé selon la revendication 3, **caractérisé en ce que**, lorsque le critère d'activité est satisfait, les données de référence sont actualisées à l'aide des données de profil sur lesquelles repose la satisfaction dudit critère.

5. Procédé selon la revendication 3 ou 4, **caractérisé en ce que** les données de référence sont déterminées en fonction du nombre de personnes dans l'unité d'infrastructure (1) et/ou du jour calendaire, des données de référence basées sur le nombre de personnes actuel et/ou le jour calendaire actuel étant utilisées pour la comparaison dans le critère d'activité.

6. Procédé selon l'une des revendications précédentes, **caractérisé en ce que** la comparaison est effectuée pour plusieurs durées et/ou pour au moins une fenêtre temporelle glissante comme durée et/ou le critère d'activité peut être ajusté par l'utilisateur en ce qui concerne le seuil de réussite de la comparaison.

7. Procédé selon l'une des revendications précédentes, **caractérisé en ce que** le critère d'activité évalue en outre des variations de valeur de consommation (21, 22) pour au moins un autre milieu de consommation, en particulier la chaleur et/ou un gaz.

8. Procédé selon l'une des revendications précédentes, **caractérisé en ce que** les capteurs d'activité (17) utilisés sont des capteurs de sol décrivant une personne (2) allongée sur un sol de l'unité d'infrastructure (1) et/ou des capteurs infrarouges pour la détection de chutes et/ou des détecteurs de mouvement et/ou des capteurs infrarouges dans des répartiteurs de frais de chauffage et/ou des capteurs d'air respirable mesurant l'air respirable dans l'unité d'infrastructure (1), en particulier la teneur en CO2, et/ou des capteurs de courant associés à des consommateurs individuels.

9. Procédé selon l'une des revendications précédentes, **caractérisé en ce qu'**une demande d'intervention est délivrée à la personne (2) comme mesure d'alarme par le biais d'un module de sortie (18), notamment un module de sortie (18) associé à l'unité d'infrastructure (1) et/ou un module de sortie (18) associé à la personne (2), une mesure d'urgence, en particulier le contact avec un service d'urgence et/ou une personne de contact, étant déclenchée seulement si la personne (2) n'effectue aucune intervention pendant une durée de fonctionnement.

10. Procédé selon l'une des revendications précédentes, **caractérisé en ce qu'**une information d'alarme est envoyée comme mesure d'alarme à une personne de contact et/ou à un service d'urgence.

11. Dispositif (13) destiné à surveiller l'activité d'au moins une personne (2) dans une unité d'infrastructure (1) alimentée en milieux de consommation comprenant au moins l'électricité et l'eau,
chaque milieu de consommation étant associé à un module de mesure (6, 7, 8) destiné à mesurer une valeur de consommation du milieu de consommation, ledit dispositif comportant :
- une interface de données (15) destinée à recevoir des données de profil qui sont fournies par les modules de mesure (6, 7, 8) et qui contiennent des variations de valeur de consommation dans le temps (21, 22) au moins pour l'électricité et l'eau pendant une durée prédéterminée,
- un module de mémorisation (12) destiné à mémoriser des données de référence qui décrivent un profil de référence décrivant une activité normale et
- un module d'évaluation (16) destiné à évaluer les données de profil afin de déterminer si un critère d'activité est satisfait au moyen d'une comparaison des données de profil à des données de référence qui décrivent un profil de comparaison faisant partie du profil de référence pour la durée afin de déterminer si et dans quelle mesure des écarts existent par rapport à l'activité normale et destiné à déclencher au moins une mesure d'alarme si le critère d'activité n'est pas satisfait en cas d'échec de la comparaison, c'est-à-dire qu'il n'indique pas une activité normale, les données de référence décrivant les corrélations croisées entre la consommation d'eau et la consommation d'électricité et l'activation d'au moins un capteur d'activité (17) et une évaluation des données de capteur à l'aide d'un critère d'urgence étant effectuées comme mesure d'alarme, une mesure d'urgence étant déclenchée en cas de satisfaction audit critère.

12. Module de mesure (6, 7, 8) destiné à l'électricité ou à l'eau comme milieu de consommation à fournir à une unité d'infrastructure (1), ledit module de mesure comprenant un dispositif (13) selon la revendication 11.
